# EUROPEAN PATENT APPLICATION

(11) **EP 2 116 608 A2**
(43) Date of publication of application: **11.11.2009**
(21) Application number: 09155937.7
(22) Date of filing: 24.03.2009
(51) Int. Cl.: C12P 7/62, C12P 7/64, C08G 63/02, C08G 63/08

(54) **Polyester synthesis**

(30) Priority: 08.05.2008 US 117189
(71) Applicant: Xerox Corporation, Rochester, New York 14644 (US)
(72) Inventor: Farrugia, Valerie M., Oakville Ontario L6H 7V8 (CA); Wosnick, Jordan, Toronto Ontario M4S 2P5 (CA); Pouw, Kristin M., Hamilton Ontario L8N 2Z7 (CA); Gerroir, Paul J., Oakville Ontario L6L 1P9 (CA)
(74) Representative: Grünecker, Kinkeldey, Stockmair & Schwanhäusser Anwaltssozietät

(57) **Abstract**

The present disclosure provides for enzymatic polymerization to produce polyester resins suitable for use in manufacturing toners.

## Description

### BACKGROUND

The present disclosure is generally directed to polyester synthesis processes and, more specifically, to processes for the enzymatic synthesis of polyester resins which may be utilized in the formation of emulsion aggregation toners.

Xerographic printing utilizes toner particles which may be produced by a variety of processes. One of the processes is an emulsion aggregation ("EA") process that forms acrylate based, e.g., styrene acrylate, toner particles and in which surfactants are used in forming the latex emulsion. See, for example, U.S. Patent No. 6,120,967, the disclosure of which is hereby incorporated by reference in its entirety, as one example of such a process. Another type of an EA process forms polyester, e.g., sodio sulfonated polyester, and which may be a surfactant-free process. See, for example, U.S. Patent No. 5,916,725, the disclosure of which is hereby incorporated by reference in its entirety, as one example of such a process.

Although acrylate based latex may be copolymerized in water via emulsion polymerization, polyester based toners utilize polyesters which may be made in bulk in a molten phase and then cooled to produce a composite, which may be later ground into a resin powder. The polyester resin powder then forms an emulsion in water via phase-inversion emulsification or solvent flashing.

In phase-inversion emulsification, the polyester resins may be sulfonated to facilitate water dispersibility of the resin particles. However, these particles are highly hygroscopic and as a result are less amenable to triboelectric charging. The phase-inversion process uses a ketone organic solvent and isopropanol to dissolve the polyester resin. The resulting solution may then be mixed with water and a base to initiate phase-inversion emulsification. The solvent may then be removed via distillation.

Solvent flashing may be used instead of phase-inversion emulsification since this process does not require sulfonate functionality in the polyester resin and, instead, dissolves the resin in a solvent, such as ethyl acetate, prior to emulsification in water. Phase-inversion emulsification may also utilize a suitable emulsifying agent, such as an anionic surfactant. Thereafter, high shear may be used to reduce the emulsion to a nanosized scale and the solvent may be evaporated (e.g., via distillation). As described above, the phase-inversion emulsification and the solvent flashing processes require extensive time, labor and laboratory equipment. In addition, the use of volatile solvents makes the processes complicated and expensive.

Combinations of amorphous and crystalline polyesters may also be used in the EA process. This resin combination provides toners with high gloss and relatively low-melting point characteristics, which allow for more energy efficient and faster printing. However, synthesis of these resins may require the use of toxic or expensive catalysts and long reaction times at high temperatures. Therefore there is a continual need for improving polyester resins synthesis.

### SUMMARY

### The present disclosure provides:

(1) A process comprising:
   contacting at least one component selected from the group consisting of dicarboxylic acids, esters, glycols, oxyacids, oxyacid esters, lactones, lactides, macrolides and combinations thereof, with an enzyme comprising a lipase, and water;
   polymerizing the at least one component to form a polyester resin; and
   recovering the polyester resin.
(2) The process of (1), wherein the lipase is obtained from an organism selected from the group consisting of Pseudomonas aeruginosa, Pseudomonas cepacia, Pseudomonas fluorescens, Aspegillus niger, Candida antarctica, Candida cylindracea, Klebsiella oxytota, and Mucor meihei.
(3) The process of (1), wherein the lipase is selected from the group consisting of lipase PA, lipase PC, lipase PF, lipase A, lipase CA, lipase B, lipase CC, lipase K, lipase MM, and combinations thereof.
(4) The process of (1), wherein the lactone is selected from the group consisting of caprolactone, pentadecalactone, hexadecenlactone, pentadecenlactone, and combinations thereof.
(5) The process of (1), further comprising contacting the at least one component with an enzyme in a solvent.
(6) The process of (1), further comprising forming the polyester resin at a temperature of from about 10°C to about 100°C for a period of time of from about 1 minute to about 24 hours.
(7) The process of (1), wherein particles comprising the polyester resin possess a glass transition temperature of from about -20°C to about 200°C, a melting point of from about 45°C to about 100°C, and a molecular weight of from about 2,000 to about 50,000.
(8) The process of (1), wherein particles comprising the polyester resin possess a size of from about 20 to about 1000 nanometers.
(9) A process comprising:
   contacting at least one component selected from the group consisting of dicarboxylic acids, esters, glycols, oxyacids, oxyacid esters, lactones, lactides, macrolides and combinations thereof, with an enzyme comprising a lipase, water, and an optional surfactant to form an emulsion;
   polymerizing the at least one component to form a polyester resin in an emulsion; and
   recovering the polyester resin.
(10) The process of (9), wherein the lipase is obtained from an organism selected from the group consisting of Pseudomonas aeruginosa, Pseudomonas cepacia, Pseudomonas fluorescens, Aspegillus niger, Candida antarctica, Candida cylindracea, Klebsiella oxytota, and Mucor meihei.
(11) The process of (9), wherein the lipase is selected from the group consisting of lipase PA, lipase PC, lipase PF, lipase A, lipase CA, lipase B, lipase CC, lipase K, lipase MM, and combinations thereof.
(12) The process of (9), wherein the lipase is immobilized on a support member selected from the group consisting of porous polymer beads, acrylic resins, cross-linked polystyrene, ceramics, and combinations thereof.
(13) The process of (9), wherein the lactone is selected from the group consisting of caprolactone, pentadecalactone, hexadecenlactone, pentadecenlactone, and combinations thereof.
(14) The process of (9), further comprising contacting the at least one component, enzyme, and water with a coalescence inhibitor selected from the group consisting of alkanes, cycloalkanes, long chain alcohols, halogenated hydrocarbons, organosilicon compounds, long-chain esters, oils, hydrophobic dye molecules, capped isocyanates, polymeric co-stabilizers, and combinations thereof.
(15) The process of (9), further comprising forming the polyester resin at a temperature of from about 20°C to about 90°C for a period of time of from about 1 minute to about 24 hours.
(16) The process of (9), wherein particles comprising the polyester resin possess a melting point of from about 55°C to about 95°C, a molecular weight of from about 3,000 to about 20,000, and a size of from about 50 to about 250 nanometers.
(17) A process comprising:
   contacting at least one component selected from the group consisting of dicarboxylic acids, esters, glycols, oxyacids, oxyacid esters, lactones, lactides, macrolides and combinations thereof, with an enzyme comprising a lipase;
   polymerizing the at least one component to form a polyester resin;
   recovering the polyester resin;
   contacting the polyester resin with at least one colorant, an optional wax, and an optional surfactant to form toner particles; and
   recovering the toner particles.
(18) The process of (17), wherein the lipase is obtained from an organism selected from the group consisting of Pseudomonas aeruginosa, Pseudomonas cepacia, Pseudomonas fluorescens, Aspegillus niger, Candida antarctica, Candida cylindracea, Klebsiella oxytota, and Mucor meihei, and the lactone is selected from the group consisting of caprolactone, pentadecalactone, hexadecenlactone, pentadecenlactone, and combinations thereof.
(19) The process of (17), wherein the lipase is selected from the group consisting of lipase PA, lipase PC, lipase PF, lipase A, lipase CA, lipase B, lipase CC, lipase K, lipase MM, and combinations thereof, and particles comprising the polyester resin possess a melting point of from about 45°C to about 100°C, a molecular weight of from about 2,000 to about 50,000, and a size of from about 20 to about 1000 nanometers.
(20) The process of (17), wherein the toner particles have a size of from about 0.1 micron to about 15 microns.

### DETAILED DESCRIPTION

The present disclosure relates to enzymatic polymerization processes for the production of resins suitable for use in the formation of toners. In embodiments, processes of the present disclosure may be utilized to produce polyester resins with organically derived catalysts.

### Resins

Any monomer or starting material suitable for preparing a latex for use in a toner may be utilized. As noted above, in embodiments the toner may be produced by enzymatic polymerization in the presence or absence of a solvent. Suitable starting materials useful in forming a latex, and thus the resulting latex particle, optionally in an emulsion, include, but are not limited to, monomers having at least two carboxyl groups, dicarboxylic acids or corresponding esters in combination with glycols, oxyacids, oxyacid esters, lactones, lactides, macrolides, combinations thereof, and the like.

In embodiments, the resin of the latex may include at least one polymer. In embodiments, at least one polymer may be from about one to about twenty and, in embodiments, from about three to about ten. Suitable resins include polyester resins, including the resins described in U.S. Patent Nos. 6,593,049 and 6,756,176, the disclosures of each of which are hereby incorporated by reference in their entirety. Suitable resins may also include a mixture of an amorphous polyester resin and a crystalline polyester resin as described in U.S. Patent No. 6,830,860, the disclosure of which is hereby incorporated by reference in its entirety.

In embodiments, the resin may be a polyester resin formed by reacting a diol with a diacid in the presence of an organic catalyst, in embodiments an enzyme. For forming a crystalline polyester, suitable organic diols include aliphatic diols with from about 2 to about 36 carbon atoms, such as 1,2-ethanediol, 1,3-propanediol, 1,4-butanediol, 1,5-pentanediol, 1,6-hexanediol, 1,7-heptanediol, 1,8-octanediol, 1,9-nonanediol, 1,10-decanediol, 1,12-dodecanediol and the like; alkali sulfo-aliphatic diols such as sodio 2-sulfo-1,2-ethanediol, lithio 2-sulfo-1,2-ethanediol, potassio 2-sulfo-1,2-ethanediol, sodio 2-sulfo-1,3-propanediol, lithio 2-sulfo-1,3-propanediol, potassio 2-sulfo-1,3-propanediol, mixture thereof, and the like. The aliphatic diol may be, for example, selected in an amount of from about 45 to about 50 mole percent of the resin, and the alkali sulfo-aliphatic diol can be selected in an amount of from about 0 to about 10 mole percent of the resin.

Examples of organic diacids or diesters selected for the preparation of the crystalline resins include oxalic acid, succinic acid, glutaric acid, adipic acid, suberic acid, azelaic acid, sebacic acid, dodecanedioic acid, phthalic acid, isophthalic acid, terephthalic acid, naphthalene-2,6-dicarboxylic acid, naphthalene-2,7-dicarboxylic acid, cyclohexane dicarboxylic acid, malonic acid and mesaconic acid, a diester or anhydride thereof; and a sulfo-p-hydroxybenzoic acid, N,N-bis(2-hydroxyethyl)-2-amino ethane sulfonate, or mixtures thereof. The organic diacid may be selected in an amount of, for example, from about 40 to about 50 mole percent of the resin, and the alkali sulfo-aliphatic diacid can be selected in an amount of from about 0 to about 10 mole percent of the resin.

Examples of crystalline resins include polyesters, polyamides, polyimides, polyolefins, polyethylene, polybutylene, polyisobutyrate, ethylene-propylene copolymers, ethylene-vinyl acetate copolymers, polypropylene, mixtures thereof, and the like. Specific crystalline resins may be polyester based, such as poly(ethylene-adipate), poly(propylene-adipate), poly(butylene-adipate), poly(pentylene-adipate), poly(hexylene-adipate), poly(octylene-adipate), poly(ethylene-succinate), poly(propylene-succinate), poly(butylene-succinate), poly(pentylene-suceinate), poly(hexylene-succinate), poly(octylene-succinate), poly(ethylene-sebacate), poly(propylene-sebacate), poly(butylene-sebacate), poly(pentylene-sebacate), poly(hexylene-sebacate), poly(octylene-sebacate), alkali copoly(5-sulfoisophthaloyl)-copoly(ethylene-adipate), alkali copoly(5-sulfoisophthaloyl)-copoly(propylene-adipate), alkali copoly(5-sulfoisophthaloyl)-copoly(butylene-adipate), alkali copoly(5-sulfo-isophthaloyl)-copoly(pentylene-adipate), alkali copoly(5-sulfo-isophthaloyl)-copoly(hexylene-adipate), alkali copoly(5-sulfo-isophthaloyl)-copoly(octylene-adipate), alkali copoly(5-sulfo-isophthaloyl)-copoly(ethylene-adipate), alkali copoly(5-sulfo-isophthaloyl)-copoly (propylene-adipate), alkali copoly(5-sulfo-isophthaloyl)-copoly(butylene-adipate), alkali copoly(5-sulfo-isophthaloyl)-copoly(pentylene-adipate), alkali copoly(5-sulfo-isophthaloyl)-copoly(hexylene-adipate), alkali copoly(5-sulfo-isophthaloyl)-copoly(octylene-adipate), alkali copoly(5-sulfoisophthaloyl)-copoly(ethylene-succinate), alkali copoly(5-sulfoisophthaloyl)-copoly(propylene-succinate), alkali copoly(5-sulfoisophthaloyl)-copoly(butylenes-succinate), alkali copoly(5-sulfoisophthaloyl)-copoly(pentylene-succinate), alkali copoly(5-sulfoisophthaloyl)-copoly(hexylene-succinate), alkali copoly(5-sulfoisophthaloyl)-copoly(octylene-succinate), alkali copoly(5-sulfo-isophthaloyl)-copoly(ethylene-sebacate), alkali copoly(5-sulfo-isophthaloyl)-copoly(propylene-sebacate), alkali copoly(5-sulfo-isophthaloyl)-copoly(butylene-sebacate), alkali copoly(5-sulfo-isophthaloyl)-copoly(pentylene-sebacate), alkali copoly(5-sulfo-isophthaloyl)-copoly(hexylene-sebacate), alkali copoly(5-sulfo-isophthaloyl)-copoly(octylene-sebacate), alkali copoly(5-sulfo-isophthaloyl)-copoly(ethylene-adipate), alkali copoly(5-sulfo-isophthaloyl)-copoly(propylene-adipate), alkali copoly(5-sulfo-isophthaloyl)-copoly(butylene-adipate), alkali copoly(5-sulfo-isophthaloyl)-copoly(pentylene-adipate), alkali copoly(5-sulfo-isophthaloyl)-copoly(hexylene-adipate), poly(octylene-adipate), wherein alkali is a metal like sodium, lithium or potassium. Examples of polyamides include poly(ethylene-adipamide), poly(propylene-adipamide), poly(butylenes-adipamide), poly(pentylene-adipamide), poly(hexylene-adipamide), poly(octylene-adipamide), poly(ethylene-succinamide), and poly(propylene-sebecamide). Examples of polyimides include poly(ethylene-adipimide), poly(propylene-adipimide), poly(butylene-adipimide), poly(pentylene-adipimide), poly(hexylene-adipimide), poly(octylene-adipimide), poly(ethylene-succinimide), poly(propylene-succinimide), and poly(butylene-succinimide).

The crystalline resin may be present, for example, in an amount of from about 5 to about 30 percent by weight of the toner components, in embodiments from about 15 to about 25 percent by weight of the toner components. The crystalline resin can possess various melting points of, for example, from about 30° C to about 120° C, in embodiments from about 50° C to about 90° C. The crystalline resin may have a number average molecular weight (Mₙ), as measured by gel permeation chromatography (GPC) of, for example, from about 1,000 to about 50,000, in embodiments from about 2,000 to about 25,000, and a weight average molecular weight (M_{W}) of, for example, from about 2,000 to about 100,000, in embodiments from about 3,000 to about 80,000, as determined by Gel Permeation Chromatography using polystyrene standards. The molecular weight distribution (M_{w}/Mₙ) of the crystalline resin may be, for example, from about 2 to about 6, in embodiments from about 2 to about 4.

Examples of diacid or diesters selected for the preparation of amorphous polyesters include dicarboxylic acids or diesters such as terephthalic acid, phthalic acid, isophthalic acid, fumaric acid, maleic acid, succinic acid, itaconic acid, succinic acid, succinic anhydride, dodecylsuccinic acid, dodecylsuccinic anhydride, glutaric acid, glutaric anhydride, adipic acid, pimelic acid, suberic acid, azelaic acid, dodecanedioic acid, dimethyl terephthalate, diethyl terephthalate, dimethylisophthalate, diethylisophthalate, dimethylphthalate, phthalic anhydride, diethylphthalate, dimethylsuccinate, dimethylfumarate, dimethylmaleate, dimethylglutarate, dimethyladipate, dimethyl dodecylsuccinate, and combinations thereof. The organic diacid or diester may be selected, for example, from about 45 to about 52 mole percent of the resin.

Examples of diols utilized in generating the amorphous polyester include 1,2-propanediol, 1,3-propanediol, 1,2-butanediol, 1,3-butanediol, 1,4-butanediol, pentanediol, hexanediol, 2,2-dimethylpropanediol, 2,2,3-trimethylhexanediol, heptanediol, dodecanediol, bis(hyroxyethyl)-bisphenol A, bis(2-hydroxypropyl)-bisphenol A, 1,4-cyclohexanedimethanol, 1,3-cyclohexanedimethanol, xylenedimethanol, cyclohexanediol, diethylene glycol, bis(2-hydroxyethyl) oxide, dipropylene glycol, dibutylene, and combinations thereof. The amount of organic diol selected can vary, and may be, for example, from about 45 to about 52 mole percent of the resin.

Catalysts which may be utilized in forming either the crystalline or amorphous polyesters include enzymes described in greater detail below.

In embodiments, an unsaturated polyester resin may be utilized as a latex resin. Examples of such resins include those disclosed in U.S. Patent No. 6,063,827, the disclosure of which is hereby incorporated by reference in its entirety. Exemplary unsaturated polyester resins include, but are not limited to, poly(propoxylated bisphenol co-fumarate), poly(ethoxylated bisphenol co-fumarate), poly(butyloxylated bisphenol co-fumarate), poly(co-propoxylated bisphenol co-ethoxylated bisphenol co-fumarate), poly(1,2-propylene fumarate), poly(propoxylated bisphenol co-maleate), poly(ethoxylated bisphenol co-maleate), poly(butyloxylated bisphenol co-maleate), poly(co-propoxylated bisphenol co-ethoxylated bisphenol co-maleate), poly(1,2-propylene maleate), poly(propoxylated bisphenol co-itaconate), poly(ethoxylated bisphenol co-itaconate), poly(butyloxylated bisphenol co-itaconate), poly(co-propoxylated bisphenol co-ethoxylated bisphenol co-itaconate), poly(1,2-propylene itaconate), and combinations thereof.

An example of a linear propoxylated bisphenol A fumarate resin which may be utilized as a latex resin is available under the trade name SPARII from Resana S/A Industrias Quimicas, Sao Paulo Brazil. Other propoxylated bisphenol A fumarate resins that may be utilized and are commercially available include GTUF and FPESL-2 from Kao Corporation, Japan, and EM181635 from Reichhold, Research Triangle Park, North Carolina and the like.

In other embodiments, oxyacids may be utilized to form polyester resins in accordance with the processes of the present disclosure. Suitable oxyacids include, but are not limited to, 3,-hydroxybutanoic acid, 4-hydroxybutanoic acid, 3-hydroxyvaleric acid, 4-hydroxyvaleric acid, 5-hydroxyvaleric acid, 6-hydroxyhexanoic acid, 10-hydroxydecanoic acid, 11-hydroxyundecanoic acid, 12-hydroxydodecanoic acid, 15-hydroxypentadecanoic acid, combinations thereof, and the like.

In other embodiments, polymers for use in forming the resins may be 6-, 12-, 13-, 16-, and 17-membered lactones or macrolides. In embodiments, suitable starting materials for forming the resins include lactones having a core based upon 16-hydroxyhexadec-9-enoic acid. Other suitable lactones include caprolactone, pentadecalactone, hexadecenlactone, pentadecenlactone, combinations thereof, and the like. The resulting polylactones, for example, poly(pentadecalactone), poly(hexadecenlactone), and the like, may have a molecular weight of about 2,000 or above, in embodiments from about 5,000 to about 50,000, in embodiments from about 10,000 to about 30,000.

### Enzymes

As noted above, in embodiments the toner may be produced by enzymatic polymerization. The enzymes utilized thus serve as a catalyst in forming the polyester resins. Suitable enzymes to form polyesters include *Pseudomonas* family lipases, such as lipases from *Pseudomonas aeruginosa* (lipase PA), *Pseudomonas cepacia* (lipase PC), *Pseudomonas fluorescens* (lipase PF), as well as lipases from *Aspegillus niger* (lipase A), *Candida antarctica* (lipase CA or lipase B), *Candida cylindracea* (lipase CC), *Klebsiella oxytoca* (lipase K), *Mucor meihei* (lipase MM), combinations thereof, and the like.

In embodiments, the lipase may be immobilized on a support member such as porous polymer beads, acrylic resins, cross-linked polystyrene, or any other suitable polymeric and/or ceramic supports within the purview of those skilled in the art.

The lipases may be obtained from the above organisms utilizing methods within the purview of those skilled in the art. In embodiments, the organisms may be grown in incubation vessels and fed with nutrients and sugars (e.g., glucose). By selecting the right growth and treatment conditions, lipases may be obtained from the organisms. In other embodiments, the lipases may be obtained from commercial sources, for example, Fluka BioChemika, Novozymes, and/or Sigma Aldrich.

The lipases utilized to catalyze formation of the polyester resins are able to operate at temperatures of from about 10° C to about 100° C, in embodiments from about 20° C to about 90° C, in other embodiments from about 45° C to about 75° C. One advantage of the immobilized lipases that distinguishes them from other conventional catalysts is they can be separated from the product and re-used.

In embodiments, the amount of lipase enzyme utilized to catalyze a reaction may be from about 0.1 % by weight to about 10 % by weight based on the starting materials used to generate the polyester resin, in embodiments from about 1 % by weight to about 6 % by weight based on the starting materials used to generate the polyester resin.

### Reaction Conditions

The starting materials utilized to form the polyester resin, for example the diacids and diols, oxyacids, and lactones described above, may be combined with the above enzymes and a polyester may be formed by an enzymatic polymerization process. The enzymatic polymerization may be performed in the presence or absence of solvents.

In the enzymatic polymerization process, the reactants may be added to a suitable reactor, such as a mixing vessel. The appropriate amount of starting materials may be optionally dissolved in a solvent, a lipase based enzyme may be added to the solution, and a polyester formed which may then be used in the production of a toner. In other embodiments the starting materials may be combined with the lipase enzyme without solvent and a polyester formed.

Where utilized, suitable solvents include, but are not limited to, water and/or organic solvents including toluene, benzene, xylene, tetrahydrofuran, combinations thereof, and the like.

Where the starting materials are in solution, the starting materials may be at a concentration of from about 10 % by weight to about 90% by weight, in embodiments from about 30 % by weight to about 60 % by weight.

Examples of the enzymatic polymerization of various starting materials are depicted below. For example the enzymatic polymerization of a dicarboxylic acid and a glycol or diol may proceed as follows:

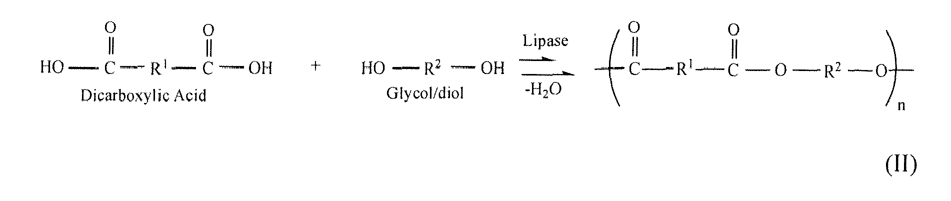

The enzymatic polymerization of an oxyacid may proceed as follows:

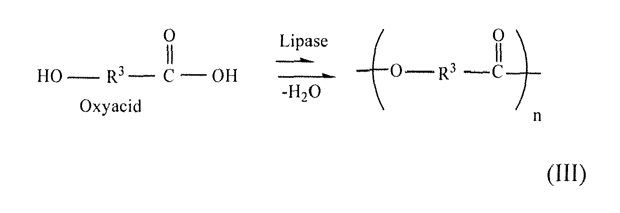

Finally, the enzymatic polymerization of a lactone may proceed as follows:

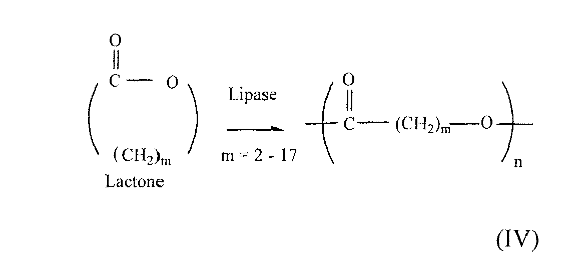

In embodiments, lactones having from about 2 to about 17 members may be catalyzed by the lipase enzyme to synthesize polylactones.

The time for the reaction may depend upon the type and amount of starting materials utilized, the amount of enzyme utilized, temperature, and the like. In embodiments, the reaction mixture may be mixed for from about 1 minute to about 72 hours, in embodiments from about 4 hours to about 24 hours, while keeping the temperature within the operational range of the lipase being used, in embodiments from about 10° C to about 100° C, in embodiments from about 20° C to about 90° C, in other embodiments from about 45° C to about 75° C.

In embodiments, the reactions illustrated by formulas (II), (III) and (IV) above may be performed directly in water.

In other embodiments a polyester emulsion may be directly produced by mixing one or more of the starting materials, enzyme, surfactant and water in a reactor to form an emulsion. More specifically, the starting materials and enzymes described above may be combined with water and one, two, or more surfactants. The surfactants may be selected from ionic surfactants and nonionic surfactants. Anionic surfactants and cationic surfactants are encompassed by the term "ionic surfactants." In embodiments, the surfactant may be utilized so that it is present in an amount of from about 0.01 % to about 5% by weight of the polyester resin, for example from about 0.75% to about 4% by weight of the resin, in embodiments from about 1% to about 3% by weight of the resin.

Examples of nonionic surfactants that can be utilized include, for example, polyacrylic acid, methalose, methyl cellulose, ethyl cellulose, propyl cellulose, hydroxy ethyl cellulose, carboxy methyl cellulose, polyoxyethylene cetyl ether, polyoxyethylene lauryl ether, polyoxyethylene octyl ether, polyoxyethylene octylphenyl ether, polyoxyethylene oleyl ether, polyoxyethylene sorbitan monolaurate, polyoxyethylene stearyl ether, polyoxyethylene nonylphenyl ether, dialkylphenoxy poly(ethyleneoxy) ethanol, available from Rhone-Poulenc as IGEPAL CA-210™, IGEPAL CA-520™, IGEPAL CA-720™, IGEPAL CO-890™, IGEPAL CO-720™, IGEPAL CO-290™, IGEPAL CA-210™, ANTAROX 890™ and ANTAROX 897™. Other examples of suitable nonionic surfactants include a block copolymer of polyethylene oxide and polypropylene oxide, including those commercially available as SYNPERONIC PE/F, in embodiments SYNPERONIC PE/F 108. Yet other examples of suitable nonionic surfactants include ethoxylates of alkyl polyethylene glycol ethers based on the C 10-Guerbet alcohol, including those commercially available as LUTENSOL^{®} AT 50 from BASF.

Anionic surfactants which may be utilized include sulfates and sulfonates, sodium dodecylsulfate (SDS), sodium dodecylbenzene sulfonate, sodium dodecylnaphthalene sulfate, dialkyl benzenealkyl sulfates and sulfonates, acids such as abitic acid available from Aldrich, NEOGEN R™, NEOGEN SC™ obtained from Daiichi Kogyo Seiyaku, combinations thereof, and the like. Other suitable anionic surfactants include, in embodiments, DOWFAX™ 2A1, an alkyldiphenyloxide disulfonate from The Dow Chemical Company, and/or TAYCA POWER BN2060 from Tayca Corporation (Japan), which are branched sodium dodecyl benzene sulfonates. Combinations of these surfactants and any of the foregoing anionic surfactants may be utilized in embodiments.

Examples of the cationic surfactants, which are usually positively charged, include, for example, alkylbenzyl dimethyl ammonium chloride, dialkyl benzenealkyl ammonium chloride, lauryl trimethyl ammonium chloride, alkylbenzyl methyl ammonium chloride, alkyl benzyl dimethyl ammonium bromide, benzalkonium chloride, cetyl pyridinium bromide, C₁₂, C₁₅, C₁₇ trimethyl ammonium bromides, halide salts of quaternized polyoxyethylalkylamines, dodecylbenzyl triethyl ammonium chloride, MIRAPOL™ and ALKAQUAT™, available from Alkaril Chemical Company, SANIZOL™ (benzalkonium chloride), available from Kao Chemicals, and the like, and mixtures thereof.

In forming the emulsions, the starting materials, enzyme, surfactant, and water may be combined utilizing any means within the purview of those skilled in the art. In embodiments, the reaction mixture may be mixed for from about 1 minute to about 72 hours, in embodiments from about 4 hours to about 24 hours, while keeping the temperature within the operational range of the lipase being used, in embodiments from about 10° C to about 100° C, in embodiments from about 20° C to about 90° C, in other embodiments from about 45° C to about 75° C.

In embodiments, a coalescence inhibitor may be added to the emulsion to aid in the formation of miniemulsions, for example, minimize the formation of large particles in the emulsion and to enhance emulsion stability by preventing coalescence and Ostwald ripening. Suitable coalescence inhibitors may include one or more of the following: hydrocarbons, including alkanes or cycloalkanes, in embodiments alkanes or cycloalkanes having at least 12 carbon atoms (such as hexadecane and/or octadecane); long chain alcohols (such as hexadecanol and/or octadecanol); halogenated hydrocarbons, organosilicon compounds, long-chain esters, oils, including vegetable oils (such as olive oil), hydrophobic dye molecules, capped isocyanates, oligomeric and/or polymeric products of polymerization, polycondensation or polyaddition, such as polymeric co-stabilizers Combinations thereof, and the like. The amount of coalescence inhibitor added to the reaction mixture may be from about 0.01% weight to about 40% weight, in embodiments from about 0.1% weight to about 10% weight. The weights of hydrophobic co-stabilizer used herein are calculated relative to the total weight of the mixture prepared in the process of the invention.

Those skilled in the art will recognize that optimization of reaction conditions, temperature, and enzyme loading can be varied to generate polyesters of various molecular weights, and that structurally related starting materials may be polymerized using comparable techniques.

In embodiments, the final water-borne polyester emulsion may be utilized to form toner particles with no further treatment. In other embodiments, if the particle size of the polyester in the emulsion is too large, the emulsion may be subjected to homogenizing or sonication to further disperse the nanoparticles and break apart nay agglomerates or loosely bound particles. Where utilized, a homogenizer, (that is, a high shear device), may operate at a rate of from about 6,000 rpm to about 10,000 rpm, in embodiments from about 7,000 rpm to about 9,750 rpm, for a period of time of from about 0.5 minutes to about 60 minutes, in embodiments from about 5 minute to about 30 minutes.

Resins thus produced may include particles possessing a size of from about 20 nanometers to about 1000 nanometers, in embodiments from about 50 nanometers to about 250 nanometers. The resins thus produced may have a glass transition temperature (Tg) of from about -20°C to about 200°C. Resins thus produced may have a melting point (Tm) of from about 45°C to about 100°C, in embodiments form about 55°C to about 95°C. The molecular weight of the polyester may be from about 2,000 to about 50,000, in embodiments from about 3,000 to about 20,000.

As noted above, enzymatic polymerization may be used in accordance with the present disclosure to directly form a polyester latex in water. During the polymerization process, water may be one of the byproducts as a result of dehydration. Thus, while a conventional polymerization reaction of the above-identified monomers may be difficult in water due to the law of mass action, enzymatic polymerization in water may circumvent this problem due to a reduction in the activation barrier for catalysis by the lipase enzyme. The lipase enzymes may remove water molecules from the reactants and create an environment similar to the gas phase for the reactants. It is also envisioned that the lipase enzyme may remove water molecules from the reactants and substitute the removed water molecules for its active site, which is adapted for electrostatic stabilization of ionic transition states and solvates these states more effectively than water.

The weight of the resulting polymers may depend, on the starting materials, reaction conditions, and the lipase enzyme being used. Higher temperatures, in embodiments about 60 °C or above, and longer reaction times of about forty eight (48) or more hours, may yield polymers with higher molecular weights.

### Toner

The resins thus produced as described above may be utilized to form toner compositions. One, two, or more toner resins may be used. In embodiments where two or more toner resins are used, the toner resins may be in any suitable ratio (e.g., weight ratio) such as for instance about 10% (first resin)/90% (second resin) to about 90% (first resin)/10% (second resin). Such toner compositions may include optional colorants, waxes, and other additives. Toners may be formed utilizing any method within the purview of those skilled in the art. In embodiments, the toner may include emulsion aggregation toners. Emulsion aggregation involves aggregation of both submicron latex and pigment particles into toner size particles, where the growth in particle size may be, for example, in embodiments from about 0.1 micron to about 15 microns.

### Surfactants

Surfactants which may be utilized in preparing resins and toners with the processes of the present disclosure include anionic, cationic, and/or nonionic surfactants. Such surfactants may find use in forming latex resins in emulsion polymerization synthesis methods, and may also find use in forming toner particles in emulsion aggregation processes, phase inversion processes, and the like.

Anionic surfactants which may be utilized include sulfates and sulfonates, sodium dodecylsulfate (SDS), sodium dodecylbenzene sulfonate, sodium dodecylnaphthalene sulfate, dialkyl benzenealkyl sulfates and sulfonates, acids such as abitic acid available from Aldrich, NEOGEN R™, NEOGEN SC™ obtained from Daiichi Kogyo Seiyaku, combinations thereof, and the like. Other suitable anionic surfactants include, in embodiments, DOWFAX™ 2A1, an alkyldiphenyloxide disulfonate from The Dow Chemical Company, and/or TAYCA POWER BN2060 from Tayca Corporation (Japan), which are branched sodium dodecyl benzene sulfonates. Combinations of these surfactants and any of the foregoing anionic surfactants may be utilized in embodiments.

Examples of nonionic surfactants include, but are not limited to alcohols, acids and ethers, for example, polyvinyl alcohol, polyacrylic acid, methalose, methyl cellulose, ethyl cellulose, propyl cellulose, hydroxyl ethyl cellulose, carboxy methyl cellulose, polyoxyethylene cetyl ether, polyoxyethylene lauryl ether, polyoxyethylene octyl ether, polyoxyethylene octylphenyl ether, polyoxyethylene oleyl ether, polyoxyethylene sorbitan monolaurate, polyoxyethylene stearyl ether, polyoxyethylene nonylphenyl ether, dialkylphenoxy poly(ethyleneoxy) ethanol, mixtures thereof, and the like. In embodiments commercially available surfactants from Rhone-Poulenc such as IGEPAL CA-210™, IGEPAL CA-520™, IGEPAL CA-720™, IGEPAL CO-890™, IGEPAL CO-720™, IGEPAL CO-290™, IGEPAL CA-210™, ANTAROX 890™ and ANTAROX 897™ can be selected.

Examples of cationic surfactants include, but are not limited to, ammoniums, for example, alkylbenzyl dimethyl ammonium chloride, dialkyl benzenealkyl ammonium chloride, lauryl trimethyl ammonium chloride, alkylbenzyl methyl ammonium chloride, alkyl benzyl dimethyl ammonium bromide, benzalkonium chloride, and C12, C15, C17 trimethyl ammonium bromides, mixtures thereof, and the like. Other cationic surfactants include cetyl pyridinium bromide, halide salts of quaternized polyoxyethylalkylamines, dodecylbenzyl triethyl ammonium chloride, and the like, and mixtures thereof. The choice of particular surfactants or combinations thereof, as well as the amounts of each to be used, is within the purview of those skilled in the art.

### Colorants

Such colorants include, for example, various known suitable colorants, such as dyes, pigments, mixtures of dyes, mixtures of pigments, mixtures of dyes and pigments, and the like, may be included in the toner. The colorant may be included in the toner in an amount of, for example, about 0.1 to about 35 percent by weight of the toner, or from about 1 to about 15 weight percent of the toner, or from about 3 to about 10 percent by weight of the toner.

As examples of suitable colorants, mention may be made of carbon black like REGAL 330^{®}; magnetites, such as Mobay magnetites MO8029™, MO8060™; Columbian magnetites; MAPICO BLACKS™ and surface treated magnetites; Pfizer magnetites CB4799™, CB5300™, CB5600™, MCX6369™; Bayer magnetites, BAYFERROX 8600™, 8610™; Northern Pigments magnetites, NP-604™, NP-608™; Magnox magnetites TMB-100™, or TMB-104™; and the like. As colored pigments, there can be selected cyan, magenta, yellow, red, green, brown, blue or mixtures thereof. Generally, cyan, magenta, or yellow pigments or dyes, or mixtures thereof, are used. The pigment or pigments are generally used as water based pigment dispersions.

Specific examples of pigments include SUNSPERSE 6000, FLEXIVERSE and AQUATONE water based pigment dispersions from SUN Chemicals, HELIOGEN BLUE L6900™, D6840™, D7080™, D7020™, PYLAM OIL BLUE™, PYLAM OIL YELLOW™, PIGMENT BLUE 1™ available from Paul Uhlich & Company, Inc., PIGMENT VIOLET 1™, PIGMENT RED 48™, LEMON CHROME YELLOW DCC 1026™, E.D. TOLUIDINE RED™ and BON RED C™ available from Dominion Color Corporation, Ltd., Toronto, Ontario, NOVAPERM YELLOW FGL™, HOSTAPERM PINK E™ from Hoechst, and CINQUASIA MAGENTA™ available from E.I. DuPont de Nemours & Company, and the like. Generally, colorants that can be selected are black, cyan, magenta, or yellow, and mixtures thereof. Examples of magentas are 2,9-dimethyl-substituted quinacridone and anthraquinone dye identified in the Color Index as CI 60710, CI Dispersed Red 15, diazo dye identified in the Color Index as CI 26050, CI Solvent Red 19, and the like. Illustrative examples of cyans include copper tetra(octadecyl sulfonamido) phthalocyanine, x-copper phthalocyanine pigment listed in the Color Index as CI 74160, CI Pigment Blue, and Anthrathrene Blue, identified in the Color Index as CI 69810, Special Blue X-2137, and the like. Illustrative examples of yellows are diarylide yellow 3,3-dichlorobenzidene acetoacetanilides, a monoazo pigment identified in the Color Index as CI 12700, CI Solvent Yellow 16, a nitrophenyl amine sulfonamide identified in the Color Index as Foron Yellow SE/GLN, CI Dispersed Yellow 33 2,5-dimethoxy-4-sulfonanilide phenylazo-4'-chloro-2,5-dimethoxy acetoacetanilide, and Permanent Yellow FGL. Colored magnetites, such as mixtures of MAPICO BLACK™, and cyan components may also be selected as colorants. Other known colorants can be selected, such as Levanyl Black A-SF (Miles, Bayer) and Sunsperse Carbon Black LHD 9303 (Sun Chemicals), and colored dyes such as Neopen Blue (BASF), Sudan Blue OS (BASF), PV Fast Blue B2G01 (American Hoechst), Sunsperse Blue BHD 6000 (Sun Chemicals), Irgalite Blue BCA (Ciba-Geigy), Paliogen Blue 6470 (BASF), Sudan III (Matheson, Coleman, Bell), Sudan II (Matheson, Coleman, Bell), Sudan IV (Matheson, Coleman, Bell), Sudan Orange G (Aldrich), Sudan Orange 220 (BASF), Paliogen Orange 3040 (BASF), Ortho Orange OR 2673 (Paul Uhlich), Paliogen Yellow 152, 1560 (BASF), Lithol Fast Yellow 0991K (BASF), Paliotol Yellow 1840 (BASF), Neopen Yellow (BASF), Novoperm Yellow FG 1 (Hoechst), Permanent Yellow YE 0305 (Paul Uhlich), Lumogen Yellow D0790 (BASF), Sunsperse Yellow YHD 6001 (Sun Chemicals), Suco-Gelb L1250 (BASF), Suco-Yellow D1355 (BASF), Hostaperm Pink E (American Hoechst), Fanal Pink D4830 (BASF), Cinquasia Magenta (DuPont), Lithol Scarlet D3700 (BASF), Toluidine Red (Aldrich), Scarlet for Thermoplast NSD PS PA (Ugine Kuhlmann of Canada), E.D. Toluidine Red (Aldrich), Lithol Rubine Toner (Paul Uhlich), Lithol Scarlet 4440 (BASF), Bon Red C (Dominion Color Company), Royal Brilliant Red RD-8192 (Paul Uhlich), Oracet Pink RF (Ciba-Geigy), Paliogen Red 3871K (BASF), Paliogen Red 3340 (BASF), Lithol Fast Scarlet L4300 (BASF), combinations of the foregoing, and the like.

### Wax

Wax dispersions may also be added during formation of a latex in an emulsion aggregation synthesis. Suitable waxes include, for example, submicron wax particles in the size range of from about 50 to about 1000 nanometers, in embodiments of from about 100 to about 500 nanometers in volume average diameter, suspended in an aqueous phase of water and an ionic surfactant, nonionic surfactant, or combinations thereof. The ionic surfactant or nonionic surfactant may be present in an amount of from about 0.1 to about 20 percent by weight, and in embodiments of from about 0.5 to about 15 percent by weight of the wax.

The wax dispersion according to embodiments of the present disclosure may include, for example, a natural vegetable wax, natural animal wax, mineral wax, and/or synthetic wax. Examples of natural vegetable waxes include, for example, carnauba wax, candelilla wax, Japan wax, and bayberry wax. Examples of natural animal waxes include, for example, beeswax, punic wax, lanolin, lac wax, shellac wax, and spermaceti wax. Mineral waxes include, for example, paraffin wax, microcrystalline wax, montan wax, ozokerite wax, ceresin wax, petrolatum wax, and petroleum wax. Synthetic waxes of the present disclosure include, for example, Fischer-Tropsch wax, acrylate wax, fatty acid amide wax, silicone wax, polytetrafluoroethylene wax, polyethylene wax, polypropylene wax, and combinations thereof.

Examples of polypropylene and polyethylene waxes include those commercially available from Allied Chemical and Baker Petrolite, wax emulsions available from Michelman Inc. and the Daniels Products Company, EPOLENE N-15 commercially available from Eastman Chemical Products, Inc., VISCOL 550-P, a low weight average molecular weight polypropylene available from Sanyo Kasel K.K., and similar materials. In embodiments, commercially available polyethylene waxes possess a molecular weight (Mw) of from about 100 to about 5000, and in embodiments of from about 250 to about 2500, while the commercially available polypropylene waxes have a molecular weight of from about 200 to about 10,000, and in embodiments of from about 400 to about 5000.

In embodiments, the waxes may be functionalized. Examples of groups added to functionalize waxes include amines, amides, imides, esters, quaternary amines, and/or carboxylic acids. In embodiments, the functionalized waxes may be acrylic polymer emulsions, for example, JONCRYL 74, 89, 130, 537, and 538, all available from Johnson Diversey, Inc, or chlorinated polypropylenes and polyethylenes commercially available from Allied Chemical, Baker Petrolite Corporation and Johnson Diversey, Inc.
The wax may be present in an amount of from about 0.1 to about 30 percent by weight, and in embodiments from about 2 to about 20 percent by weight of a toner including a wax, optionally in a resin utilized to form such a toner.

### Toner Preparation

The toner particles may be prepared by any method within the purview of one skilled in the art. Although embodiments relating to toner particle production are described below with respect to emulsion-aggregation processes, any suitable method of preparing toner particles may be used, including chemical processes, such as suspension and encapsulation processes disclosed in U.S. Patent Nos. 5,290,654 and 5,302,486, the disclosures of each of which are hereby incorporated by reference in their entirety. In embodiments, toner compositions and toner particles may be prepared by aggregation and coalescence processes in which small-size resin particles are aggregated to the appropriate toner particle size and then coalesced to achieve the final toner-particle shape and morphology.

In embodiments, toner compositions may be prepared by emulsion-aggregation processes, such as a process that includes aggregating a mixture of an optional colorant, an optional wax and any other desired or required additives, and emulsions including the resins described above, optionally in surfactants as described above, and then coalescing the aggregate mixture. A mixture may be prepared by adding a colorant and optionally a wax or other materials, which may also be optionally in a dispersion(s) including a surfactant, to the emulsion, which may be a mixture of two or more emulsions containing the resin. The pH of the resulting mixture may be adjusted by an acid such as, for example, acetic acid, nitric acid or the like. In embodiments, the pH of the mixture may be adjusted to from about 4 to about 5. Additionally, in embodiments, the mixture may be homogenized. If the mixture is homogenized, homogenization may be accomplished by mixing at about 600 to about 4,000 revolutions per minute. Homogenization may be accomplished by any suitable means, including, for example, an IKA ULTRA TURRAX T50 probe homogenizer.

Following the preparation of the above mixture, an aggregating agent may be added to the mixture. Any suitable aggregating agent may be utilized to form a toner. Suitable aggregating agents include, for example, aqueous solutions of a divalent cation or a multivalent cation material. The aggregating agent may be, for example, polyaluminum halides such as polyaluminum chloride (PAC), or the corresponding bromide, fluoride, or iodide, polyaluminum silicates such as polyaluminum sulfosilicate (PASS), and water soluble metal salts including aluminum chloride, aluminum nitrite, aluminum sulfate, potassium aluminum sulfate, calcium acetate, calcium chloride, calcium nitrite, calcium oxylate, calcium sulfate, magnesium acetate, magnesium nitrate, magnesium sulfate, zinc acetate, zinc nitrate, zinc sulfate, zinc chloride, zinc bromide, magnesium bromide, copper chloride, copper sulfate, and combinations thereof. In embodiments, the aggregating agent may be added to the mixture at a temperature that is below the glass transition temperature (Tg) of the resin.

The aggregating agent may be added to the mixture utilized to form a toner in an amount of, for example, from about 0.1% to about 8% by weight, in embodiments from about 0.2% to about 5% by weight, in other embodiments from about 0.5% to about 5% by weight, of the resin in the mixture. This provides a sufficient amount of agent for aggregation.

In order to control aggregation and coalescence of the particles, in embodiments the aggregating agent may be metered into the mixture over time. For example, the agent may be metered into the mixture over a period of from about 5 to about 240 minutes, in embodiments from about 30 to about 200 minutes, although more or less time may be used as desired or required. The addition of the agent may also be done while the mixture is maintained under stirred conditions, in embodiments from about 50 rpm to about 1,000 rpm, in other embodiments from about 100 rpm to about 500 rpm.

The particles may be permitted to aggregate and/or coalesce until a predetermined desired particle size is obtained. A predetermined desired size refers to the desired particle size to be obtained as determined prior to formation, and the particle size being monitored during the growth process until such particle size is reached. Samples may be taken during the growth process and analyzed, for example with a Coulter Counter, for average particle size. The aggregation/coalescence thus may proceed by maintaining the elevated temperature, or slowly raising the temperature to, for example, from about 40°C to about 100°C, and holding the mixture at this temperature for a time from about 0.5 hours to about 6 hours, in embodiments from about hour 1 to about 5 hours, while maintaining stirring, to provide the aggregated particles. Once the predetermined desired particle size is reached, then the growth process is halted. In embodiments, the predetermined desired particle size is within the toner particle size ranges mentioned above.

The growth and shaping of the particles following addition of the aggregation agent may be accomplished under any suitable conditions. For example, the growth and shaping may be conducted under conditions in which aggregation occurs separate from coalescence. For separate aggregation and coalescence stages, the aggregation process may be conducted under shearing conditions at an elevated temperature, for example of from about 40°C to about 90°C, in embodiments from about 45°C to about 80°C, which may be below the glass transition temperature of the resin as discussed above.

Following aggregation to the desired particle size, the particles may then be coalesced to the desired final shape, the coalescence being achieved by, for example, heating the mixture to a temperature of from about 65°C to about 105°C, in embodiments from about 70°C to about 95°C, which may be at or above the glass transition temperature of the resin, and/or increasing the stirring, for example to from about 400 rpm to about 1,000 rpm, in embodiments from about 500 rpm to about 800 rpm. Higher or lower temperatures may be used, it being understood that the temperature is a function of the resins used for the binder. Coalescence may be accomplished over a period of from about 0.1 to about 9 hours, in embodiments from about 0.5 to about 4 hours.

After aggregation and/or coalescence, the mixture may be cooled to room temperature, such as from about 20°C to about 25°C. The cooling may be rapid or slow, as desired. A suitable cooling method may include introducing cold water to a jacket around the reactor. After cooling, the toner particles may be optionally washed with water, and then dried. Drying may be accomplished by any suitable method for drying including, for example, freeze-drying.

### Other Additives

Further optional additives which may be combined with a toner include any additive to enhance the properties of toner compositions. Included are surface additives, color enhancers, etc. Surface additives that can be added to the toner compositions after washing or drying include, for example, metal salts, metal salts of fatty acids, colloidal silicas, metal oxides, strontium titanates, combinations thereof, and the like, which additives are each usually present in an amount of from about 0.1 to about 10 weight percent of the toner, in embodiments from about 0.5 to about 7 weight percent of the toner. Examples of such additives include, for example, those disclosed in U.S. Patent Nos. 3,590,000, 3,720,617, 3,655,374 and 3,983,045, the disclosures of each of which are hereby incorporated by reference in their entirety. Other additives include zinc stearate and AEROSIL R972^{®} available from Degussa. The coated silicas of U.S. Patent No. 6,190,815 and U.S. Patent No. 6,004,714, the disclosures of each of which are hereby incorporated by reference in their entirety, can also be selected in amounts, for example, of from about 0.05 to about 5 percent by weight of the toner, in embodiments from about 0.1 to about 2 percent by weight of the toner. These additives can be added during the aggregation or blended into the formed toner product.

In embodiments, an aggregation method may be utilized whereby the latex resin particles are formed, and then aggregated and coalesced with a colorant and optional wax and other additives to form toner particles. In embodiments, such a process may include combining from about 100 grams to about 500 grams of at least one resin, in other embodiments from about 150 grams to about 250 grams of at least one resin, with from about 0.5 grams to about 50 grams of a colorant, in embodiments from about 1 grams to about 10 grams of a colorant and from about 50 grams to about 150 grams of a wax, in embodiments from about 75 grams to about 125 grams of a wax. The components may be combined in a suitable solvent, such as a ketone, alcohol, ester, combinations thereof, and the like, and heated to a temperature of from about 50° C to about 90° C, in embodiments from about 60° C to about 80° C, in some embodiments about 70° C.

The solution thus produced may then be combined with a separate aqueous solution, in embodiments including a stabilizer and a surfactant in deionized water, to produce an emulsion of the latex having the colorant. In embodiments the two solutions may be subjected to homogenization by mixing at high shear, in embodiments from about 8,000 rpm to about 12,000 rpm, in other embodiments from about 9,000 rpm to about 11,000 rpm, in other embodiments about 10,000 rpm, for from about 15 minutes to about 60 minutes, in embodiments from about 20 minutes to about 45 minutes, in embodiments about 30 minutes. The reaction mixture may then be distilled at a temperature of from about 60° C to about 100° C, in embodiments from about 70° C to about 90° C, in some embodiments about 80° C, for a period of time from about 1 hour to about 3 hours, in embodiments about 2 hours. The resulting emulsion may be stirred for a period of time from about 12 hours to about 18 hours, with the resulting latex possessing a colorant isolated by filtering, centrifuging, decanting, combinations thereof, and the like.

Toner particles produced utilizing a latex of the present disclosure may have a size of about 1 micron to about 20 microns, in embodiments about 2 microns to about 15 microns, in embodiments about 3 microns to about 7 microns, in some embodiments about 5.9 microns. Toner particles of the present disclosure may have a circularity of from about 0.9 to about 0.99, in embodiments from about 0.92 to about 0.98, in some embodiments about 0.94.

### Uses

The enzymatic polymerization synthesis according to the present disclosure may be used to prepare resins for use in subsequent synthesis of emulsion aggregation toners either in the presence or absence of solvents. Crystalline or amorphous polyester resins with a range of thermodynamic and chemical properties may be produced. The disclosed enzymatic synthesis also provides for reduced reaction times and energy costs, since lipase based synthesis occurs at temperatures from about 10°C to about 100°C, in embodiments for a period of time as short as about four hours, whereas processes with conventional catalysts may require temperatures above about 150 °C and may take at least eight or more hours.

As noted above, in embodiments lipase enzymes may be used to catalyze ring-opening polymerization of lactones, even large-ring lactones, such as pentadecalactone and hexadecenlactone, to obtain corresponding polyesters thereof. Large-ring lactones may be resistant to traditional chemical polymerization processes due to low ring strain of these compounds. Enzymatic polymerization may thus produce polyester structures which are not available through conventional lactone polymerization processes. More specifically, polymerization of pentadecalactone may produce a crystalline polymer with a melting point of at least 79 °C, depending on molecular weight, and polymerization of hexadecenlactone may produce a polymer having a much lower melting point of 57 °C. It is envisioned that copolymerization of these monomers, as well as other lactones, such as pentadecenlactone, caprolactone, derivatives of caprolactane and similar compounds, may be used to produce polymers of any suitable crystallinity and melting/glass transition temperatures.

In addition, lactones suitable for enzymatic polymerization are readily available, making the starting material relatively inexpensive since the large-ring lactones are widely used in other industries, for example fragrance, food additives, and the like.

Further, the lipase enzymes are reusable, thereby reducing the costs associate with their use. Thus, the enzymatic polymerization processes of the present disclosure, including the formation of water-borne polyester emulsions described above, are environmentally friendly by reducing the energy required for production and reducing the use of solvents by avoiding phase inversion and/or solvent flashing steps.

### Imaging

Imaging methods are also envisioned with the toners disclosed herein. Such methods include, for example, some of the above patents mentioned above and U.S. Patent Nos. 4,265,990, 4,584,253 and 4,563,408, the entire disclosures of each of which are incorporated herein by reference. The imaging process includes the generation of an image in an electronic printing magnetic image character recognition apparatus and thereafter developing the image with a toner composition of the present disclosure. The formation and development of images on the surface of photoconductive materials by electrostatic means is well known. The basic xerographic process involves placing a uniform electrostatic charge on a photoconductive insulating layer, exposing the layer to a light and shadow image to dissipate the charge on the areas of the layer exposed to the light, and developing the resulting latent electrostatic image by depositing on the image a finely-divided electroscopic material, for example, toner. The toner will normally be attracted to those areas of the layer, which retain a charge, thereby forming a toner image corresponding to the latent electrostatic image. This powder image may then be transferred to a support surface such as paper. The transferred image may subsequently be permanently affixed to the support surface by heat. Instead of latent image formation by uniformly charging the photoconductive layer and then exposing the layer to a light and shadow image, one may form the latent image by directly charging the layer in image configuration. Thereafter, the powder image may be fixed to the photoconductive layer, eliminating the powder image transfer. Other suitable fixing means such as solvent or overcoating treatment may be substituted for the foregoing heat fixing step.

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" refers to a temperature of from about 20 ° C to about 25° C.

### EXAMPLES

### EXAMPLE 1

Production of a crystalline polyester resin. About 10.22 grams of pentadecalactone (from Lancaster) was dissolved in about 15 ml of toluene in a reaction vessel, which was previously dried by standing over activated 4A molecular sieves. The solution was stirred until the pentadecalactone was substantially dissolved in a dry atmosphere. About 50 mg, or about 0.5 % by weight, of *Candida antarctica* (lipase B) disposed on acrylic resin, which was previously activated by drying in vacuum for about twelve hours, was added to the solution. The resulting mixture was stirred for about four (4) hours at about 70 °C to produce a viscous solution.

The viscous solution was poured out of the reaction vessel and allowed to cool to room temperature producing a white, spongy material. The resulting material was purified by redissolution in about 100 ml of toluene and then filtered through a coarse sintered glass funnel. The filtered toluene solution was slowly added to about 1400 ml of methanol with vigorous stirring producing a white, fibrous precipitate. The precipitate was filtered and dried in a vacuum at about 45 °C for a time of from about twelve hours to about 20 hours, providing about 8.21 grams of poly(pentadecalactone) having a total number-average molecular weight of about 3,000 grams/mol and a melting point (Tm) of about 79 °C.

The above synthesis of poly(pentadecalactone) by polymerization of pentadecalactone via *Candida antarctica* (lipase B) in toluene may be summarized as follows:

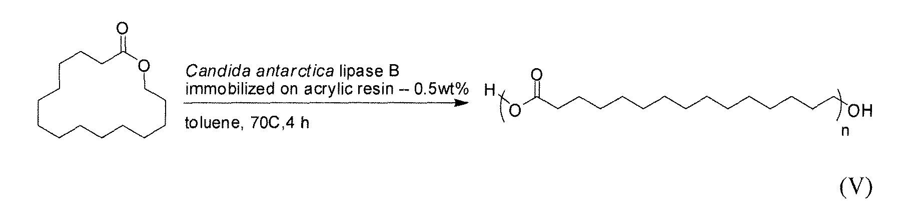

### EXAMPLE 2

Production of a polyester resin emulsion with *Pseudomonas cepacia* (lipase PC). About 12 grams of pentadecalactone (50 mmol) was combined with about 0.5 grams of hexadecane (from Sigma-Aldrich) and about 50 grams of 1% by weight solution of LUTENSOL^{®} AT 50 (a nonionic surfactant including ethoxylates of alkyl polyethylene glycol ethers based on the C 10-Guerbet alcohol commercially available from BASF) in water. The mixture was stirred for about 1 hour at about 300 rpm at about 45 °C. The mixture was then homogenized for about 120 seconds at about 6,000 rpm. About 0.25 grams of about 24 % by weight *Pseudomonas cepacia* (lipase PC) in water and about 25 grams of about 1% by weight of LUTENSOL^{®} AT 50 in water were added to the homogenized mixture. The mixture was reacted in a sealed round bottom flask, which was placed in an oil bath for about 24 hours at approximately 60 °C and was stirred at about 300 rpm.

After about 24 hours, a portion of the reaction mixture was bottled for stability studies and the remaining portion was dried in a vacuum oven.

The above synthesis of poly(pentadecalactone) by polymerization of pentadecalactone via *Pseudomonas cepacia* (lipase PC) in water may be summarized as follows:

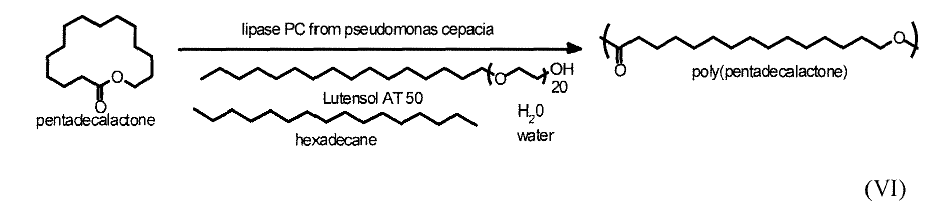

### EXAMPLE 3

Production of a polyester resin emulsion with *Candida antarctica* (lipase CA). About 48 grams of pentadecalactone (200 mmol) was combined with about 2 grams of hexadecane (from Sigma-Aldrich) and about 200 grams of about 1% by weight solution of LUTENSOL^{®} AT 50 (from BASF) in water. The mixture was stirred for about 1 hour at about 300 rpm at about 45°C. The mixture was then homogenized for about 120 seconds at about 6,000 rpm. About 0.24 grams of about 10% by weight *Candida antartica* (lipase CA) immobilized on cross-linked acrylic resin was added to the homogenized mixture. The mixture was then reacted in a closed reactor with a cold condenser for water reflux for approximately 120 hours at about 70 °C and stirred at about 130 rpm. The reactor was insulated to prevent heat loss.

After about 72 hours, the reaction mixture was beginning to solidify and formed a paste which had a solids content of about 30 % by weight. The mixture was then re-emulsified with about 2% per polymer mass TAYCA POWER BN2060 surfactant (branched sodium dodecyl benzene sulphonate) to reduce particle size and solids content. About 10 grams of the mixture was homogenized in about 150 grams of water with about 0.34 grams of TAYCA POWER BN2060 by stirring at about 6,000 rpm for approximately 30 minutes at about 95 °C.

The emulsion was then further homogenized by stirring at about 6,000 rpm for about 40 minutes as the mixture was cooled to room temperature. The resulting stable emulsion was checked for solids content and particle size. A sample of the resulting emulsion was dried and submitted for gel permeation chromatography for comparison with the paste produced after 72 hours to ensure that homogenization with water had no hydrolyzing effect on the polymeric product.

To increase the solids content, the mixture was re-emulsified with about 2 % per polymer mass TAYCA POWER BN2060 surfactant (branched sodium dodecyl benzene sulphonate). About 10 grams of the mixture was homogenized in about 23.75 grams of water with about 0.34 grams of TAYCA POWER BN2060 at about 6000 rpm for about 30 minutes at about 95 °C.

The above synthesis of poly(pentadecalactone) by polymerization of pentadecalactone via *Candida antarctica* (lipase CA) in water may be summarized as follows:

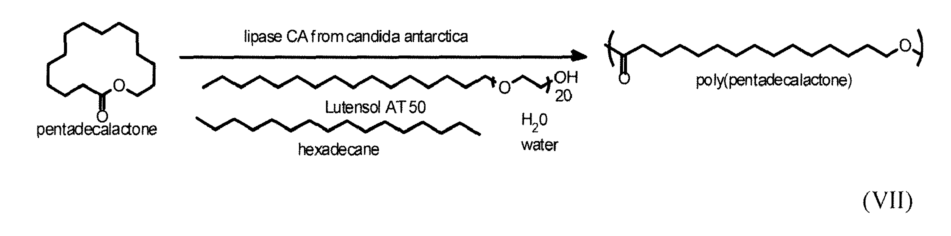

### EXAMPLE 4

Analysis and characterization. The polyester emulsions of Examples 2 and 3 were analyzed for polymer molecular weights, molecular number and polydispersity, by gel permeation chromatography using polystyrene standards as a calibration curve (to calculate Mw). The melting points of the polymers were measured by a differential scanning calorimeter (DSC). Particle sizes were measured by dynamic light scattering using a Microtrac UPA 150. Particles were examined using a JEOL 6300F scanning electron microscope (SEM) where a droplet of the emulsion was placed on a fragment of a silicon wafer and allowed to dry before being coated with a thin, sputtered film of Pt/Pd.

It was observed from the SEM that particles in the miniemulsion produced by the process of the present disclosure were generally spherical. The average particle size was from about 100 nm to about 150 nm. Observed results are summarized in Table 1 below.

**Table 1**

| | | | | | | | | |
|---|---|---|---|---|---|---|---|---|
| SAMPLE ID | Rxn Temp (°C) | Rxn Time (h) | Melting Point (°C) | Mw | Mn | D | Particle (nm) (%) | Solids Content % |
| Example 2 | 60 | 24 | 91.2 | 5,500 | 3,100 | 1.79 | 190 | 13.35 |
| Example 3 | 70 | 100 | 74.9/ 90.3 | Does not dissolve in THF, predicting high Mw | | | 113 | 30.00 |

The samples produced had excellent particle size distribution and the unimodal particles produced were of acceptable size for aggregation/coalescence in the production of toners. The molecular weight of these polymer species was greater than about 10,000.

## Claims

1. A process comprising:
contacting at least one component selected from the group consisting of dicarboxylic acids, esters, glycols, oxyacids, oxyacid esters, lactones, lactides, macrolides and combinations thereof, with an enzyme comprising a lipase, and water;
polymerizing the at least one component to form a polyester resin; and
recovering the polyester resin.

2. A process as in claim 1, wherein the lipase is obtained from an organism selected from the group consisting of Pseudomonas aeruginosa, Pseudomonas cepacia, Pseudomonas fluorescens, Aspegillus niger, Candida antarctica, Candida cylindracea, Klebsiella oxytota, and Mucor meihei.

3. A process as in claim 1, wherein the lipase is selected from the group consisting of lipase PA, lipase PC, lipase PF, lipase A, lipase CA, lipase B, lipase CC, lipase K, lipase MM, and combinations thereof.

4. A process as in claim 1, wherein the lactone is selected from the group consisting of caprolactone, pentadecalactone, hexadecenlactone, pentadecenlactone, and combinations thereof.

5. A process as in claim 1, further comprising contacting the at least one component with an enzyme in a solvent.

6. A process as in claim 1, further comprising forming the polyester resin at a temperature of from about 10°C to about 100°C for a period of time of from about 1 minute to about 24 hours.

7. A process comprising:
contacting at least one component selected from the group consisting of dicarboxylic acids, esters, glycols, oxyacids, oxyacid esters, lactones, lactides, macrolides and combinations thereof, with an enzyme comprising a lipase, water, and an optional surfactant to form an emulsion;
polymerizing the at least one component to form a polyester resin in an emulsion; and
recovering the polyester resin.

8. A process as in claim 7, wherein the lipase is obtained from an organism selected from the group consisting of Pseudomonas aeruginosa, Pseudomonas cepacia, Pseudomonas fluorescens, Aspegillus niger, Candida antarctica, Candida cylindracea, Klebsiella oxytota, and Mucor meihei.

9. A process as in claim 7, wherein the lipase is selected from the group consisting of lipase PA, lipase PC, lipase PF, lipase A, lipase CA, lipase B, lipase CC, lipase K, lipase MM, and combinations thereof.

10. A process as in claim 7, wherein the lipase is immobilized on a support member selected from the group consisting of porous polymer beads, acrylic resins, cross-linked polystyrene, ceramics, and combinations thereof.

11. A process as in claim 7, wherein the lactone is selected from the group consisting of caprolactone, pentadecalactone, hexadecenlactone, pentadecenlactone, and combinations thereof.

12. A process as in claim 7, further comprising contacting the at least one component, enzyme, and water with a coalescence inhibitor selected from the group consisting of alkanes, cycloalkanes, long chain alcohols, halogenated hydrocarbons, organosilicon compounds, long-chain esters, oils, hydrophobic dye molecules, capped isocyanates, polymeric co-stabilizers, and combinations thereof.

13. A process comprising:
contacting at least one component selected from the group consisting of dicarboxylic acids, esters, glycols, oxyacids, oxyacid esters, lactones, lactides, macrolides and combinations thereof, with an enzyme comprising a lipase;
polymerizing the at least one component to form a polyester resin;
recovering the polyester resin;
contacting the polyester resin with at least one colorant, an optional wax, and an optional surfactant to form toner particles; and
recovering the toner particles.

14. A process as in claim 13, wherein the lipase is obtained from an organism selected from the group consisting of Pseudomonas aeruginosa, Pseudomonas cepacia, Pseudomonas fluorescens, Aspegillus niger, Candida antarctica, Candida cylindracea, Klebsiella oxytota, and Mucor meihei, and the lactone is selected from the group consisting of caprolactone, pentadecalactone, hexadecenlactone, pentadecenlactone, and combinations thereof.

15. A process as in claim 13, wherein the lipase is selected from the group consisting of lipase PA, lipase PC, lipase PF, lipase A, lipase CA, lipase B, lipase CC, lipase K, lipase MM, and combinations thereof, and particles comprising the polyester resin possess a melting point of from about 45°C to about 100°C, a molecular weight of from about 2,000 to about 50,000, and a size of from about 20 to about 1000 nanometers.
